# EUROPEAN PATENT APPLICATION

(11) **EP 3 243 484 A1**
(43) Date of publication of application: **15.11.2017**
(21) Application number: 15877103.0
(22) Date of filing: 28.12.2015
(51) Int. Cl.: A61F 2/24, A61B 5/107

(54) **LEAFLET SIZER**

(30) Priority: 06.01.2015 JP 2015001193
(71) Applicant: Japanese Organization for Medical Device Development, Inc., Tokyo 103-0023 (JP); Toho University, Tokyo 143-8540 (JP)
(72) Inventor: OZAKI, Shigeyuki, Tokyo 152-0032 (JP)
(74) Representative: Peters, Sebastian Martinus
(86) International application number: PCT/JP2015/086525
(87) International publication number: WO 2016/111231

(57) **Abstract**

The present invention provides a leaflet sizer comprising first and second commissure indication parts (11, 13) to be contacted with two commissures of a cardiac valve, the first and second commissure indication parts being movable relative to each other.

## Description

### TECHNICAL FIELD

The present invention relates to a leaflet sizer for measuring the size of a cardiac valve. The leaflet sizer is used at the time of, for example, aortic valvuloplasty or aortic valve reconstruction where for a cardiac valve, a leaflet material is formed from an artificial membrane or a biological membrane.

### BACKGROUND

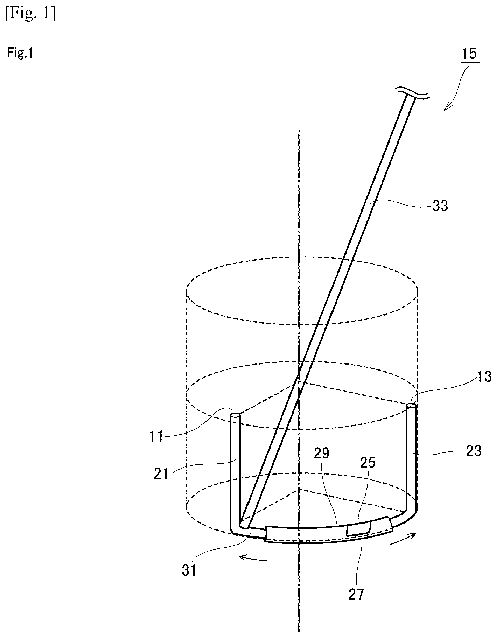

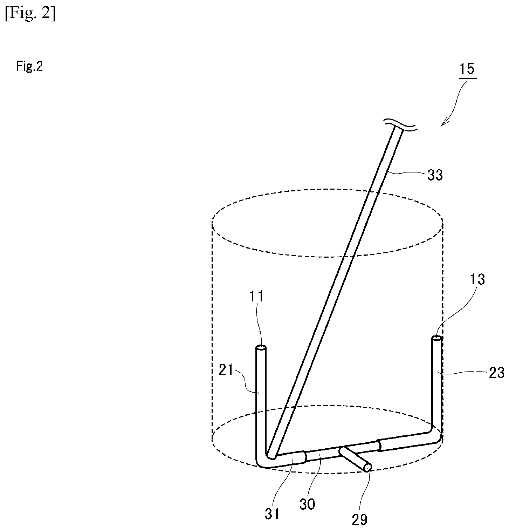

JP 5106019 B2 (Patent Literature 1 described below) discloses a leaflet forming instrument. This leaflet forming instrument has plural leaflet sizers and a template. An example of the leaflet sizer is illustrated in Fig. 2 of this literature. That is, the leaflet sizer in this literature has sizer blocks and handles, and an arc surface formed by cutting a column by an angle corresponding to a center angle of commissure parts is formed in the sizer block (Claim 2). A stylus portion that positions both end parts of the arc surface in the commissure parts is provided in the sizer block. As illustrated in Fig. 1 of the literature, the leaflet forming instrument disclosed in this literature has a shape in which the sizer blocks in plural sizes are respectively connected to the handles.

Aortic valvuloplasty is introduced in, for example, Ozaki, S. "Aortic valvuloplasty using patient's own cardiac membrane" JINKOU ZOUKI Vol. 39, Issue 3, 2010, p.p. 157-161 (Non Patent Literature 1). In this literature, as described above, a leaflet sizer having sizer blocks and handles is also used (for example, see Fig. 2).

### CITATION LIST

### Patent Literature

Patent Literature 1: JP 5106019 B2

### Non Patent Literature

Non Patent Literature 1: Ozaki, S. "Aortic valvuloplasty using patient's own cardiac membrane" JINKOU ZOUKI Vol. 39, Issue 3, 2010, p.p. 157-161

### SUMMARY OF THE INVENTION

### TECHNICAL PROBLEM

Aortic valvuloplasty is a revolutionary medical approach. Meanwhile, the above leaflet forming instrument requires leaflet sizers in plural sizes. Even when one leaflet forming instrument includes plural leaflet sizers, about one or two leaflet sizer is used for one procedure in practice. Therefore, there is a problem that a large number of leaflet sizers become useless.

The leaflet forming instrument is originally a disposable medical instrument. However, since a large number of leaflet sizers are not used at the time of a procedure as described above, there is a possibility that the leaflet forming instrument is re-used.

Further, in the conventional leaflet sizer, with a sizer head formed in a fan shape, the handle is attached to a pivot part of the fan at an angle. Therefore, when the conventional leaflet sizer is inserted deep into the artery, there is a problem that the handle is touched to the anterior wall of aorta and the sizer is not easily inserted into a far part.

An object of the present invention is to provide a leaflet sizer capable of preventing a situation in which the leaflet sizer is re-used. The present invention also relates to a leaflet sizer to be easily inserted into the aorta at the time of a procedure and hence the size of a leaflet is easily measured.

### SOLUTION TO PROBLEM

A first aspect of the present invention relates to one or a small number of leaflet sizer (or a leaflet sizer set) having functions of the conventional plural leaflet sizers. The leaflet sizer has first and second commissure pointing portions 11, 13 to be applied to two commissure parts in a cardiac valve. Thereby, the leaflet sizer can determine the size of the cardiac valve. In the leaflet sizer 15 of the first aspect of the present invention, relative positions of the first and second commissure pointing portions 11, 13 are variable.

As an example of the leaflet sizer in which the relative positions of the first and second commissure pointing portions 11, 13 are variable, the leaflet sizer has a first support portion 21 supporting the first commissure pointing portion 11, and the first support portion 21 is extendable and retractable. In this case, the leaflet sizer may further have a second support portion 23 supporting the second commissure pointing portion 13. The second support portion 23 is also extendable and retractable.

As an example of the leaflet sizer in which the relative positions of the first and second commissure pointing portions 11, 13 are variable, the leaflet sizer further has an indicator display portion 25 that displays an indicator corresponding to a distance of the two commissure parts when the first support portion 21 is extended or retracted.

As an example of an extending and retracting function in the leaflet sizer in which the relative positions of the first and second commissure pointing portions 11, 13 are variable, the leaflet sizer has a leaflet sizer main body having a housing portion 27 for slidably housing the first support portion.

As a preferred example of the leaflet sizer, the leaflet sizer further has a leaflet sizer main body 31 connected to the first and second commissure pointing portions 11, 13, and a handle 33 connected to the leaflet sizer main body 31, and the handle 33 exists at a position in the vicinity of one of the first and second commissure pointing portions 11, 13.

In another embodiment, the leaflet sizer of the present invention may have a first arm 51, a second arm 52, and a coupling support point portion 53. The first arm 51 and the second arm 52 cross each other around intermediate parts thereof. The coupling support point portion 53 openably and closably couples the first and second arms 51, 52 at a crossing point of the first and second arms 51, 52. In this embodiment, the first commissure pointing portion 11 is provided in a leading end of the first arm 51, and the second commissure support portion 13 is provided in a leading end of the second arm 52. In such a way, the relative positions of the first and second commissure pointing portions 11, 13 may be variable.

In still another embodiment, the leaflet sizer of the present invention may have a base portion 61, a rod 62, a first arm 63, and a second arm 64. The rod 62 is inserted into the base portion 61. This rod 62 is capable of going forward and backward in the base portion 61 by being guided by the base portion 61. The first arm 63 and the second arm 64 are coupled to this base portion 61. The first and second arm portions 63 are opened and closed linked with actions of the rod 62 going forward and backward in the base portion 61. In this embodiment, the first commissure pointing portion 11 is provided in a leading end of the first arm 63, and the second commissure support portion 13 is provided in a leading end of the second arm 64. In such a way, the relative positions of the first and second commissure pointing portions 11, 13 may be variable.

A second aspect of the present invention relates to a leaflet sizer set 17 having plural leaflet sizer heads 15a, 15b, 15c. The leaflet sizer heads 15a, 15b, 15c are medical instruments respectively having first and second commissure pointing portions 11a, 13a to be applied to two commissure parts in a cardiac valve for determining the size of the cardiac valve. Relative positions of the first and second commissure pointing portions are different respectively in the plural leaflet sizer heads 15a, 15b, 15c. That is, the leaflet sizer set 17 has the leaflet sizer heads in plural sizes. The plural leaflet sizers respectively have coupling holes 41a, 41b, 41c for coupling the plural leaflet sizer heads, and the plural leaflet sizer heads are integrated by a restraining body 43 passing through the coupling holes.

### ADVANTAGEOUS EFFECTS OF INVENTION

The leaflet sizer of the first aspect of the present invention is one or a small number of leaflet sizer having the functions of the conventional plural leaflet sizers. Therefore, the number of the leaflet sizers to be unused at all but disposed can be reduced. As a result, re-use of the leaflet sizer can be prevented.

In the leaflet sizer in which the handle is in the vicinity of one of the commissure support portions, the handle 33 is placed at the position in the vicinity of the aortic wall in comparison to the conventional sizer. Thus, when the sizer is inserted into the aorta, the handle is not easily touched to the anterior wall of aorta. As a result, the sizer is easily inserted into a far part of the aorta.

In the leaflet sizer of the second aspect of the present invention, the plural sizers are integrated. Thus, even in a case where part of the sizers has been utilized, it is difficult to dispose only the part. Therefore, re-use of the leaflet sizer can be prevented.

### BRIEF DESCRIPTION OF THE DRAWINGS

Fig. 1 is a concept view illustrating an example of a leaflet sizer of the present invention.
Fig. 2 is a concept view illustrating an example of the leaflet sizer of the present invention.
Fig. 3 is a concept view illustrating an example of the leaflet sizer of the present invention.
Fig. 4 is a concept view illustrating an example of the leaflet sizer of the present invention.
Fig. 5 is a perspective view illustrating an example of an outer appearance of a crossing openable and closable type leaflet sizer.
Fig. 6 is an enlarged view illustrating a state where the crossing openable and closable type leaflet sizer is closed.
Fig. 7 is an enlarged view illustrating a state where the crossing openable and closable type leaflet sizer is opened.
Fig. 8 is a schematic view illustrating an action range of the crossing openable and closable type leaflet sizer.
Fig. 9 is a perspective view illustrating a state where a rod openable and closable type leaflet sizer is closed.
Fig. 10 is a perspective view illustrating a state where the rod openable and closable type leaflet sizer is opened.
Fig. 11 is a schematic view illustrating an action range of the rod openable and closable type leaflet sizer.

### DESCRIPTION OF EMBODIMENTS

Hereinafter, modes for carrying out the present invention will be described with using the drawings. The present invention is not limited to the modes to be described below but also includes corrections of the following modes appropriately done by those skilled in the art in an obvious range.

A first aspect of the present invention is one or a small number of leaflet sizer having functions of the conventional plural leaflet sizers. The leaflet sizer is a known medical instrument as disclosed in Patent Literature 1 (JP 5106019 B2) and Non Patent Literature 1 (Ozaki, S. "Aortic valvuloplasty using patient's own cardiac membrane" JINKOU ZOUKI Vol. 39, Issue 3, 2010, p.p. 157-161) as described above.

Fig. 1 is a concept view illustrating an example of the leaflet sizer of the present invention. As illustrated in Fig. 1, the leaflet sizer has first and second commissure pointing portions 11, 13 to be applied to two commissure parts in a cardiac valve. The first and second commissure pointing portions 11, 13 correspond to the stylus portion in Patent Literature 1 (JP 5106019 B2). The first and second commissure pointing portions 11, 13 are parts to be inserted into a cut valve ring part at the time of a procedure for respectively pointing the commissure parts positioned on both ends. By the first and second commissure pointing portions 11, 13, the leaflet sizer can determine the size of a cardiac valve. In the leaflet sizer 15 of the first aspect of the present invention, relative positions of the first and second commissure pointing portions 11, 13 are variable.

As an example of the leaflet sizer in which the relative positions of the first and second commissure pointing portions 11, 13 are variable, the leaflet sizer has a first support portion 21 supporting the first commissure pointing portion 11, and the first support portion 21 is extendable and retractable. In this case, the leaflet sizer may further have a second support portion 23 supporting the second commissure pointing portion 13. The second support portion 23 may also be extendable and retractable. The phrase "extendable and retractable" means that the relative positions of the first and second commissure pointing portions 11, 13 are variable.

As an example of the leaflet sizer in which the relative positions of the first and second commissure pointing portions 11, 13 are variable, the leaflet sizer further has an indicator display portion 25 that displays an indicator corresponding to a distance of the two commissure parts when the first support portion 21 is extended or retracted. For example, in a housing portion 27, a device that measures an end part of a leaflet sizer main body 31 existing in the housing portion 27 may be provided, and by measuring a position of the end part by this device, the relative positions of the first and second commissure pointing portions 11, 13 may be grasped and the grasped relative positions may be displayed on the indicator display portion 25.

As an example of an extending and retracting function in the leaflet sizer in which the relative positions of the first and second commissure pointing portions 11, 13 are variable, the leaflet sizer has the leaflet sizer main body 31 having the housing portion 27 for slidably housing the first support portion 21. By the leaflet sizer main body 31 sliding inside the housing portion 27, the relative positions of the first and second commissure pointing portions 11, 13 can be changed.

As a preferred example of the leaflet sizer, the leaflet sizer further has the leaflet sizer main body 31 connected to the first and second commissure pointing portions 11, 13, and a handle 33 connected to the leaflet sizer main body 31. The handle 33 exists at a position in the vicinity of one of the first and second commissure pointing portions 11, 13. In a case where the x coordinates of the first and second commissure pointing portions 11, 13 are 0 (x₁) and x₂, respectively, and the x coordinate of the handle 33 is x₃, x₃ may preferably be 0 or more and 0.4x₂ or less, or 0 or more and 0.3x₂ or less, or 0 or more and 0.2x₂ or less, or 0 or more and 0.1x₂ or less. When the handle is displaced from the first commissure pointing portion 11, the sizer is more balanced. Thus, x₃ may be 0.05x₂ or more and 0.3x₂ or less, or 0.1x₂ or more and 0.2x₂ or less. A part of the handle 33 in contact with the housing portion 27 or the leaflet sizer main body 31 may be the center of the housing body 27 or the leaflet sizer main body 31. However, in that case, the handle is preferably inclined toward any of the first and second commissure pointing portions 11, 13.

As a preferred example of the leaflet sizer, a mark 29 exists in a center part of an edge part. This mark is useful for marking at the time of a procedure.

In an example illustrated in Fig. 1, the sizer main body 31 exists in an edge part of a column. The example has a structure in which the first and second support portions 21, 23 are bent upward from both ends (or from the vicinity of both the ends) of the edge part. In this example, the first commissure pointing portion 11 and the first support portion 21 exist on the left side of the center of a main body part in Fig. 1, and the second commissure pointing portion 13 and the second support portion 23 exist on the right side of the center of the main body part in Fig. 1. Parts of the first and second support portions 21, 23 existing in the edge part are partly or entirely housed in the housing portion 27. This example has a tubular body having an inner diameter which is the same as or larger than the outer shape of the edge part in such a manner that the housing portion 27 can house edge parts of the first and second support portions 21, 23.

Although not particularly shown in the figures, as an example of the extending and retracting function in the leaflet sizer in which the relative positions of the first and second commissure pointing portions 11, 13 are variable, the example being different from the above example, at least the edge part of any of or both of the first support portion 21 and the second support portion is extendable and retractable. In the example, the edge part has a spring structure, a bellows structure, and an elastic body. An example of the elastic body is rubber.

For example, when a force is applied to the handle 33, the first support body 21 housed in the housing portion 27 is moved in the left direction (or the right direction) in Fig. 1, and the relative positions of the first and second commissure pointing portions 11, 13 are extended. As a preferable mode of the example illustrated in Fig. 1, when the first support body 21 is moved in the left direction or the right direction in Fig. 1, the second support body 23 is moved in the opposite direction to the first support body 21. This can be realized by a mechanism in which when the first support body 21 is moved, the second support body 23 is moved in the opposite direction.

In an example illustrated in Fig. 2, any of or both of the first support portion 21 and the second support portion is hollow, and a coupling portion 30 is inserted into the hollow part thereof. Any of or both of the first support portion 21 and the second support portion can slide on the coupling part. In such a way, the relative positions of the first and second commissure pointing portions 11, 13 can be adjusted. In this example, a bar for the mark 29 for pointing the center extends from the center of the coupling portion 30. In the example illustrated in Fig. 2, elements are described like a bar shape. However, in the example illustrated in Fig. 2, since the first and second support portions 21, 23 are positioned on the outer sides of the coupling portion 30, the first and second support portions 21, 23 can have a smooth structure (rather bold structure). Therefore, this example can easily have a solid shape with which an actual procedure is easily performed. Specifically, the first and second support portions 21, 23 can be formed in a shape along the inner wall of blood vessel.

A second aspect of the present invention relates to a leaflet sizer set 17 having plural leaflet sizer heads 15a, 15b, 15c. The leaflet sizer heads 15a, 15b, 15c are medical instruments respectively having first and second commissure pointing portions 11a, 13a to be applied to two commissure parts in a cardiac valve for determining the size of the cardiac valve. Relative positions of the first and second commissure pointing portions are different respectively in the plural leaflet sizer heads 15a, 15b, 15c. That is, the leaflet sizer has the leaflet sizer heads in plural sizes. The plural leaflet sizers respectively have coupling holes 41a, 41b, 41c for coupling the plural leaflet sizer heads, and the plural leaflet sizer heads are integrated by a restraining body 43 passing through the coupling holes.

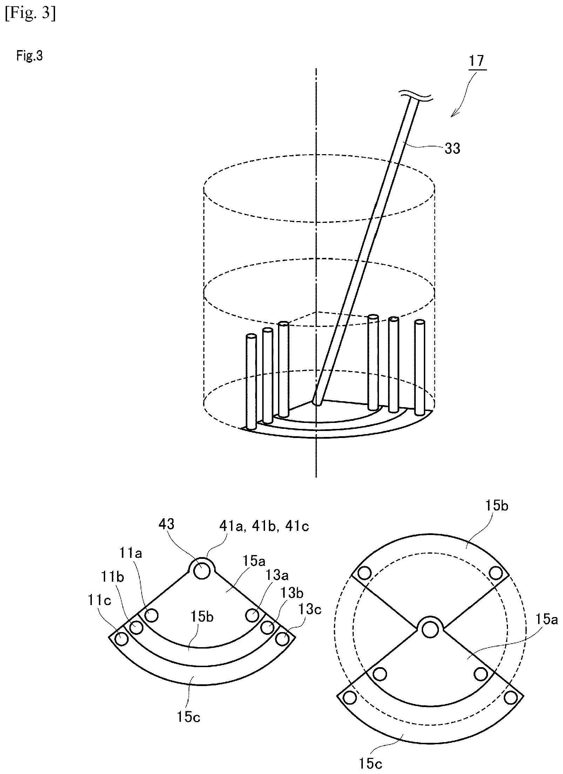

Fig. 3 is a concept view illustrating the leaflet sizer. In this example, the plural leaflet sizer heads 15a, 15b, 15c are connected rotatably about the restraining body 43. In an upper part of the restraining body, a handle 33 is formed. In a procedure, when the leaflet sizer head (for example, 15b) determined as proper by a practitioner is selected, the leaflet sizer head 15b is rotated and moved about the restraining body 43 by using the coupling hole 41b. Then, only the desired leaflet sizer head 15b can be taken out. The remaining leaflet sizer heads are also axially fixed to the restraining body 43 via the coupling holes 41a, 43c. The remaining leaflet sizer heads 15a, 15c act to press the artery and measure and test the leaflet size at the time of an actual procedure.

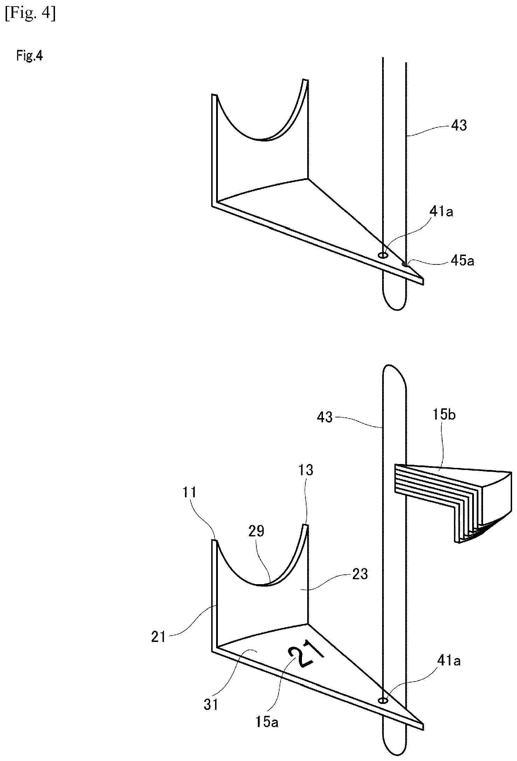

Fig. 4 is a concept view illustrating the leaflet sizer. The leaflet sizer illustrated in Fig. 4 has plural leaflet sizer heads 15a, 15b, 15c. The leaflet sizer heads 15a, 15b, 15c respectively have first and second commissure pointing portions 11a, 13a to be applied to two commissure parts in a cardiac valve. Relative positions of the first and second commissure pointing portions are different respectively in the plural leaflet sizer heads 15a, 15b, 15c. That is, the leaflet sizer has the leaflet sizer heads in plural sizes. The plural leaflet sizers respectively have coupling holes 41a for coupling the plural leaflet sizer heads, and the plural leaflet sizer heads are integrated by a restraining body 43 passing through the coupling holes. In the example of Fig. 4, the size is written on the leaflet sizer head, so that the leaflet size is found in actual measurement. In this example, the leaflet sizer heads respectively have fixing holes 45a, and by letting one end of the loop shaped restraining body 43 pass through the fixing holes 45a, the restraining body 43 can be utilized as a handle.

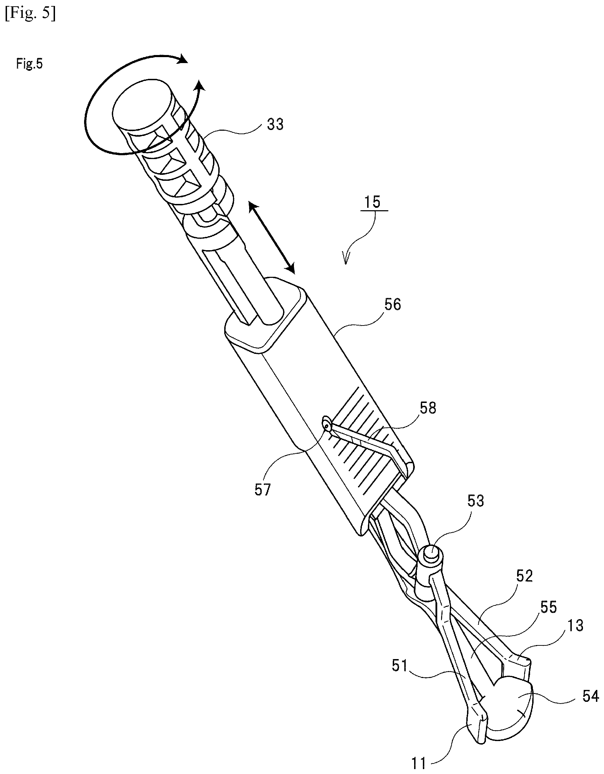

Next, a leaflet sizer according to another embodiment will be described with reference to Figs. 5, 6, 7, and 8. The leaflet sizer illustrated in Figs. 5 to 8 is called as the "crossing openable and closable type leaflet sizer" in the description of the present application.

As illustrated in Fig. 5, the crossing openable and closable type leaflet sizer has first and second arms 51, 52 crossing each other. A first commissure pointing portion 11 is provided in a leading end of the first arm 51, and a second commissure pointing portion 13 is provided in a leading end of the first arm 62. A coupling support point portion 53 is formed at a crossing point of the first arm 51 and the second arm 52. This coupling support point portion 53 couples both the arms in such a manner that the first arm 51 and the second arm 52 are openable and closable. For example, the coupling support point portion 53 may be formed by a pin, passing through the crossing point of the first arm 51 and the second arm 52 or the like. The phrase "openably and closably" means that relative positions of the leading ends of the first and second arms 51, 52 (that is, the first and second commissure pointing portions 11, 13) are variable.

In the present embodiment, the leaflet sizer has a head 54 between the first commissure pointing portion 11 and the first commissure pointing portion 13. As illustrated in Fig. 5, in a state where the first and second commissure pointing portions 11, 13 are closed, these pointing portions 11, 13 are preferably abutted with a side surface of the head 54. This head 54 is attached to a leading end of a third arm 55. The third arm 55 is coupled to the first arm 51 and the second arm 52 by the coupling support point portion 53. In the present embodiment, the third arm 55 is basically immovable. That is, the first arm 51 and the second arm 52 are opened and closed so as to be moved to the left or the right side of the third arm 55 in an immovable state with the coupling support point portion 53 as a support point. Thereby, relative positions of the first arm 51 (first commissure pointing portion 11) and the second arm 52 (second commissure pointing portion 13) can be changed with the third arm 55 (head 54) as the center.

Further, as illustrated in Fig. 5, the crossing openable and closable type leaflet sizer includes a handle 33 and a guide 56. The handle 33 serves as a gripping portion when the practitioner holds the leaflet sizer. Part of this handle 33 is inserted inside the guide 56. A screw thread is formed in the part of the handle 33 entering the guide 56 and fitted to a screw hole formed inside the guide 56. Therefore, by screwing the handle 33 in the fixed direction, the part of the handle 33 can be brought deeply inside the guide 56. On the other hand, by screwing the handle 33 in the opposite direction, the part of the handle 33 can be pulled out from the inside of the guide 56.

As illustrated in Fig. 5, the first arm 51, the second arm 52, and the third arm 55 are inserted inside the guide 56 from the opposite side of the handle 33. That is, as illustrated in the figure, the other ends of the first to third arms 51, 52, 55 opposite to the leading ends in which the pointing portions 11, 13 and the head 54 are provided are inserted inside the guide 56. The other ends of the first to third arms 51, 52, 55 are coupled to the part of the handle 33. Further, at least parts of the first and second arms 51, 52 inserted inside this guide 56 are made of a flexible material to be curved by applying a pressing force. The entire first and second arms 51, 52 can also be made of a flexible material. Therefore, when the part of the handle 33 is brought deeply inside the guide 56 by screwing the handle 33, in accordance with the pressing force, the first and second arms 51, 52 are curved inside the guide 56.

Further, as illustrated in Fig. 5, projecting slide pins 57 are formed in the parts of the first and second arms 51, 52 inserted into the guide 56. Rails 58 for guiding the slide pins 57 are formed in the guide 56. In the example illustrated in Fig. 5, the rail 58 is a cut (slit) formed in the guide 56. However, the rail 58 may be a groove formed in the guide 56. The rail 58 is provided so as to be inclined at a predetermined angle with respect to the direction in which the handle 56 goes forward and backward. Further, although omitted in the figure, the rail 58 for guiding the slide pin 57 formed in the first arm 51 and a rail (not shown) for guiding a slide pin (not shown) formed in the second arm 52 are inclined in the opposite directions, that is, the crossing directions. Therefore, when the handle 33 is gradually brought into the guide 56, the first arm 51 and the second arm 52 are guided by the rails 58, so that the relative positions thereof are gradually brought apart from each other. In such a way, by biasing in the directions in which relative positions of the other ends of the first and second arms 51, 52 are brought apart, the relative positions of the first and second commissure pointing portions 11, 13 provided in the leading ends are also brought apart with the coupling support point portion 53 as a support point. Conversely, in order to bring the relative positions of the first and second commissure pointing portions 11, 13 close, an operation which is opposite to the above operation may be performed. Thereby, by a simple operation of screwing the handle 33, the relative positions of the first and second commissure pointing portions 11, 13 can be changed.

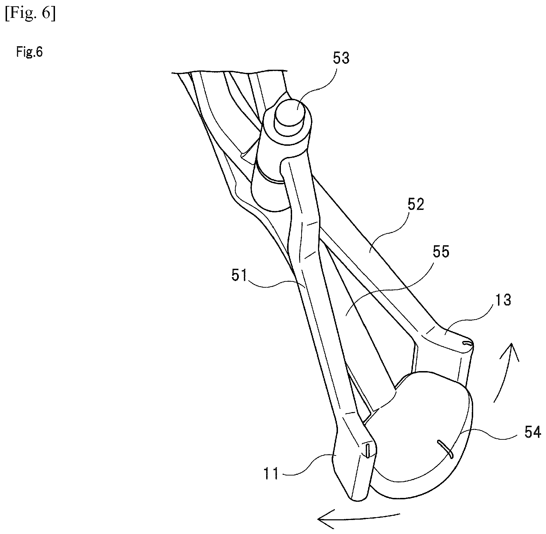

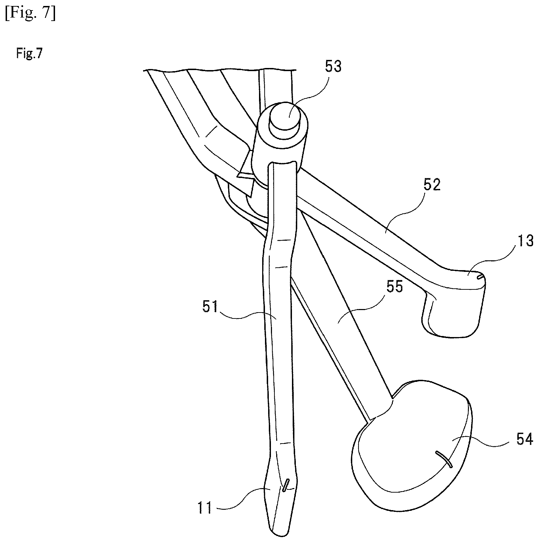

Fig. 6 illustrates a state where the leading ends of the first and second arms 51, 52, that is, the first and second commissure pointing portions 11, 13 are closed. Fig. 7 illustrates a state where the leading ends of the first and second arms 51, 52, that is, the first and second commissure pointing portions 11, 13 are opened. The first and second arms 51, 52 are gradually spread from the closed state illustrated in Fig. 6 to the opened state illustrated in Fig. 7. Therefore, by applying to commissure parts of a cardiac valve of a patient while adjusting a positional relationship of the first and second commissure pointing portions 11, 13, the size of the cardiac valve can be measured.

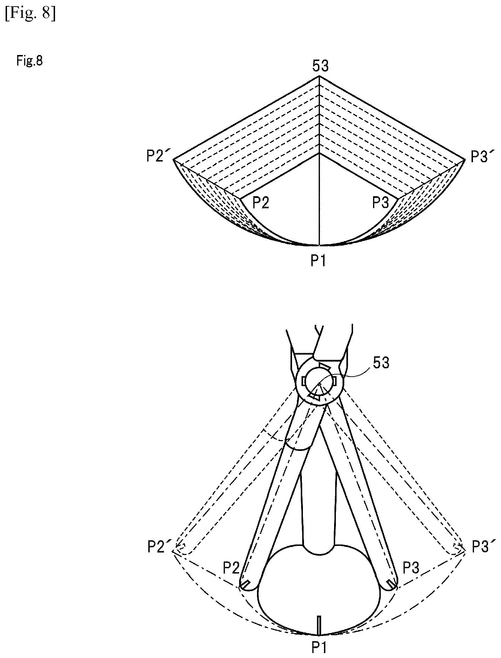

Fig. 8 schematically illustrates a state where a relation of relative positions of the first commissure pointing portion 11, the second commissure pointing portion 13, and the head 54 is changed in accordance with opening and closing of the first and second arms 51, 52. In Fig. 8, a peak position of the head 54 is denoted by the reference sign P1, a peak position of the first commissure support portion 11 is denoted by the reference sign P2, and a peak position of the second commissure pointing portion 13 is denoted by the reference sign P3. As illustrated in Fig. 8, in the crossing openable and closable type leaflet sizer, when the first and second arms 51, 52 are spread, the first and second commissure pointing portions 11, 13 are moved on the circumference of a circle centered at the coupling support point portion 33. That is, the first commissure support portion 11 is moved from P2 to P2', a distance from P2 to the coupling support point portion 33 and a distance from P2' to the coupling support point portion 33 are equal. The same is applied to the second commissure support portion 13. In the crossing openable and closable type leaflet sizer, the peak position P1 of the head 54 is immovable. When the size of a leaflet of a patient is measured by using the crossing openable and closable type leaflet sizer, there is a need for paying attention to such a characteristic of the relationship of the relative positions of P1, P2, and P3.

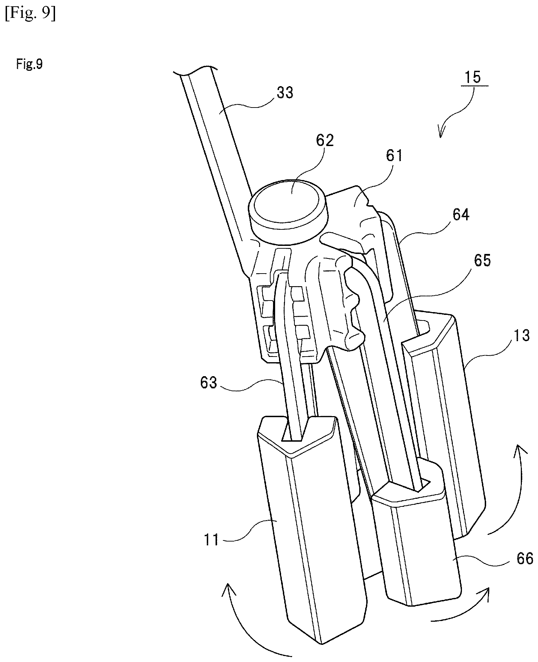

Next, a leaflet sizer according to still another embodiment will be described with reference to Figs. 9, 10, and 11. The leaflet sizer illustrated in Figs. 9 to 11 is called as the "rod openable and closable type leaflet sizer" in the description of the present application.

As illustrated in Fig. 9, the rod openable and closable type leaflet sizer has a base portion 61 for supporting a rod 62 so that the rod is capable of going forward and backward. This base portion 61 is fixed to a leading end of a bar shaped handle 33. The base portion 61 has a through hole passing in the up and down direction inside thereof. The rod 62 is inserted into the through hole. The rod 62 goes forward and backward in the up and down direction by being guided by the base portion 61.

A first arm 63 and a second arm 64 are coupled outside the base portion 61. A first commissure pointing portion 11 and a second commissure pointing portion 13 are respectively attached to leading ends of the first and second arms 63, 63 on the opposite side of ends coupled to the base portion 61. Further, in the present embodiment, a third arm 65 is coupled outside the base portion 61, and this third arm 65 is positioned between the first arm 63 and the second arm 64. A head 66 is attached to a leading end of the third arm 65.

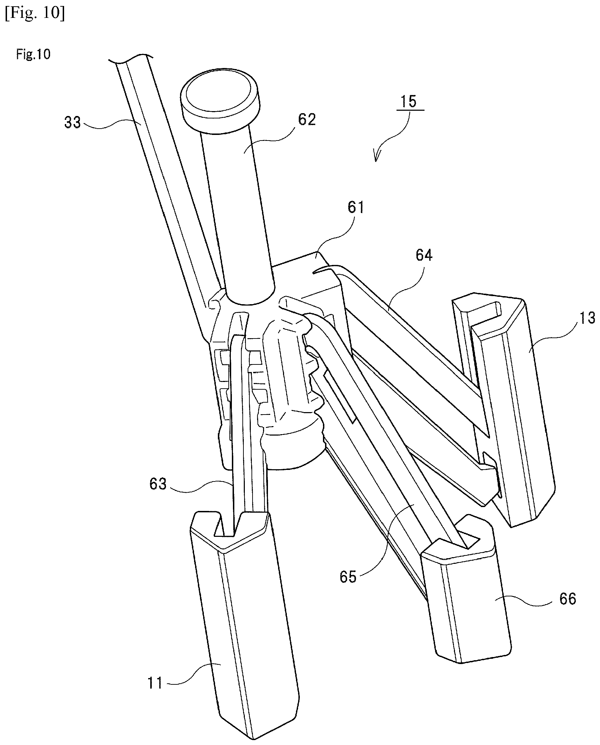

The rod openable and closable type leaflet sizer also has a linking mechanism (not shown) that links the rod 62 and the first to third arms 63, 64, 65. Such a linking mechanism is not particularly limited but can be appropriately designed by those skilled in the art. That is, as illustrated in Figs. 9 and 10, the linking mechanism links forward and backward actions of the rod 62 and opening and closing actions of the first to third arms 63, 64, 65. For example, as illustrated in Fig. 8, in a state where the rod 62 is pushed downward with respect to the base portion 61, the first to third arms 63, 64, 65 are closed. On the other hand, in a state where the rod 62 is brought upward with respect to the base portion 61, the first to third arms 63, 64, 65 are opened. By opening the first to third arms 63, 64, 65, relative positions of the first commissure pointing portion 11 and the second commissure pointing portion 13 are brought apart. In such a way, by letting the rod 62 go forward and backward, an open and close state of the first to third arms 63, 64, 65 can be freely adjusted. Therefore, by applying to commissure parts of a cardiac valve of a patient while adjusting a positional relationship of the first and second commissure pointing portions 11, 13, the size of the cardiac valve can be measured.

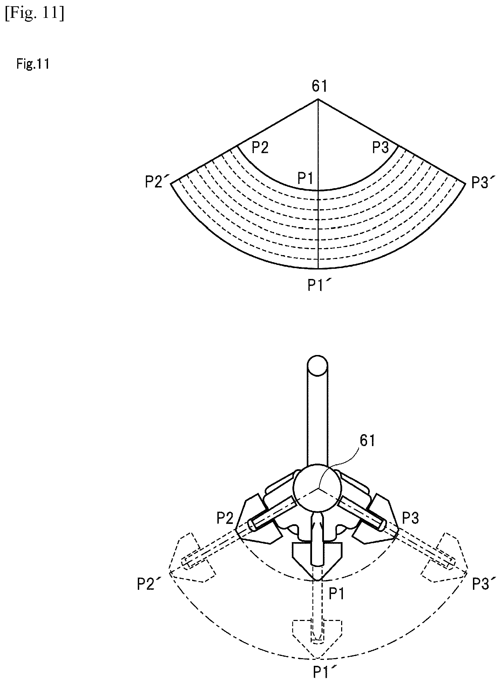

Fig. 11 schematically illustrates a state where relative positions of the first commissure pointing portion 11, the second commissure pointing portion 13, and the head 66 are changed in the rod openable and closable type leaflet sizer. In Fig. 11, a peak position of the head 66 is denoted by the reference sign P1, a peak position of the first commissure support portion 11 is denoted by the reference sign P2, and a peak position of the second commissure pointing portion 13 is denoted by the reference sign P3. As illustrated in Fig. 11, in the rod openable and closable type leaflet sizer, P1, P2, P3 are positioned on the circumference centered at a coupling support point portion 33. When the first to third arms 63, 64, 65 are spread, the positions P1, P2, P3 are changed to P1', P2', P3' in such a manner that the radius of a circle centered at the base portion 11 is extended. In the rod openable and closable type leaflet sizer, extension width of P1, P2, P3 are all uniform. That is, a distance from P1 to P1', a distance from P2 to P2', and a distance from P3 to P3' are always equal. In such a way, by using the rod openable and closable type leaflet sizer, the practitioner can measure the size of the leaflet assuming similar fan shapes.

Next, an example of aortic valve reconstruction of forming an aortic valve by using a leaflet material formed by a patient's own cardiac membrane will be described. In this case, after the sternum is exposed, median sternotomy is performed. After that, an incision is made in the cardiac membrane. In a state where the heart is exposed, extracorporeal circulation is performed with an artificial heart and an artificial lung. After a cardiac arrest, the aortic valve is exposed. Meanwhile, the cardiac membrane acquired in advance is extended and fixed by a thread and in that state, soaked into a tissue fixative solution (for example, a solution containing glutaraldehyde).

Next, part of three leaflets of the aortic valve to be removed is cut out. The leaflet sizer is applied to the cut part, and the size of the cut leaflet is measured (based on a distance between commissure parts). The cardiac membrane is taken out from the tissue fixative solution. Based on a template corresponding to the leaflet sizer (of the size corresponding to the commissure parts of the cut leaflet), a line is drawn on the cardiac membrane. The cardiac membrane is cut and a leaflet material is obtained. The leaflet material is soaked into for example a normal saline solution.

Next, a leaflet is formed by using the obtained leaflet material. Basic tasks of specific procedures are the same as JP 5106019 B2. That is, with an indicator formed at a position corresponding to a center point of the leaflet as a mark, a suture is placed on a position pointed by the indicator and the center point is stitched up. With this center point as a basis, stitching is made toward the commissure parts. In a lower end region of a leaflet base portion 25, intervals of a needle are preferably short.

### INDUSTRIAL APPLICABILITY

The present invention can be utilized in the field of medical instruments.

### REFERENCE SIGNS LIST

- 11:: First commissure pointing portion
- 13:: Second commissure pointing portion
- 15:: Leaflet sizer
- 21:: First pointing portion
- 25:: Indicator display portion
- 27:: Housing portion
- 31:: Leaflet sizer main body
- 33:: Handle
- 51:: First arm
- 52:: Second arm
- 53:: Coupling support point portion
- 61:: Base portion
- 62:: Rod portion
- 63:: First arm
- 64:: Second arm

## Claims

1. A leaflet sizer (15) for determining the size of the cardiac valve comprising:
first and second commissure pointing portions (11, 13) to be applied to two commissure parts in a cardiac valve,
wherein relative positions of the first and second commissure pointing portions are variable.

2. The leaflet sizer according to claim 1, comprising:
a first support portion (21) supporting the first commissure pointing portion,
wherein the first support portion is extendable and retractable.

3. The leaflet sizer according to claim 2, further comprising:
an indicator display portion (25) that displays an indicator corresponding to a distance of the two commissure parts when the first support portion (21) is extended or retracted.

4. The leaflet sizer according to claim 2, comprising:
a leaflet sizer main body having a housing portion (27) for slidably housing the first support portion (21).

5. The leaflet sizer according to claim 1, further comprising:
a leaflet sizer main body (31) connected to the first and second commissure pointing portions (11, 13); and
a handle (33) connected to the leaflet sizer main body (31),
wherein the handle (33) exists at a position in the vicinity of one of the first and second commissure pointing portions (11, 13).

6. The leaflet sizer according to claim 1, further comprising:
a first arm (51);
a second arm (52); and
a coupling support point portion (53) openably and closably coupling the first and second arms (51, 52) at a crossing point of the first and second arms (51, 52),
wherein the first commissure pointing portion (11) is provided in a leading end of the first arm (51), and
the second commissure support portion (13) is provided in a leading end of the second arm (52).

7. The leaflet sizer according to claim 1, further comprising:
a base portion (61);
a rod (62) to be inserted into the base portion (61), the rod being capable of going forward and backward inside the base portion (61); and
first and second arms (63, 64) coupled to the base portion (61) to be opened and closed in accordance with forward and backward movement of the rod (62),
wherein the first commissure pointing portion (11) is provided in a leading end of the first arm (63), and
the second commissure support portion (13) is provided in a leading end of the second arm (64).

8. A leaflet sizer set (17) comprising:
plural leaflet sizer heads (15a, 15b, 15c) having first and second commissure pointing portions (11a, 11b, 11c, 13a, 13b, 13c) to be applied to two commissure parts in a cardiac valve, the leaflet sizer heads for determining the size of the cardiac valve,
wherein relative positions of the first and second commissure pointing portions are different respectively in the plural leaflet sizer heads (15a, 15b, 15c),
the plural leaflet sizers respectively have coupling holes (41a, 41b, 41c) for coupling the plural leaflet sizers, and
the plural leaflet sizers are integrated by a restraining body (43) passing through the coupling holes.
